# EUROPEAN PATENT APPLICATION

(11) **EP 4 597 504 A1**
(43) Date of publication of application: **06.08.2025**
(21) Application number: 25153351.9
(22) Date of filing: 22.01.2025
(51) Int. Cl.: G16B 20/30, G16B 25/10, G16B 30/10

(54) **METHOD AND SYSTEM FOR IDENTIFYING NUCLEOMODULINS INDICATIVE OF ALTERED GENE EXPRESSION**

(30) Priority: 31.01.2024 IN 202421006325
(71) Applicant: Tata Consultancy Services Limited, Maharashtra (IN)
(72) Inventor: BOSE, Chandrani, 411057 Pune, Maharashtra (IN); KORGAONKAR, Sania Ghanashyam, 411057 Pune, Maharashtra (IN); ANAND, Swadha, 411028 Pune, Maharashtra (IN); MANDE, Sharmila Shekhar, 411028 Pune, Maharashtra (IN)
(74) Representative: Goddar, Heinz J.

(57) **Abstract**

This disclosure relates generally to a method and system for indicating gene expression changes in the host through identification of a category of microbial effector proteins called 'Nucleomodulins' (NMs). State-of-the-art methods target to fix the impaired cellular functions due to altered gene expression or altered protein expression. However, targeting NMs which are bacterial effector proteins is not yet achieved. The disclosed method provides identification of NMs in the biological sample of the host. Further, the biological sample is analyzed to identify pre-defined set of NMs or an unknown NMs through a plurality of ways. On of the way include constructing and utilizing a knowledgebase comprising NMs identified through curated literature search engines known to cause altered gene expression. Further, functional annotation of the NMs is performed by computationally analysing the corresponding protein sequences. Finally, the NMs are targeted using a nuclear constructs for controlling altered gene expression.

## Description

### CROSS-REFERENCE TO RELATED APPLICATIONS AND PRIORITY

The present application claims priority from Indian patent application no. 202421006325, filed on January 31, 2024.

### TECHNICAL FIELD

The disclosure herein generally relates to identification of biomarkers and, more particularly, to identify biomarkers indicative of altered gene expression.

### BACKGROUND

Changes in gene expression can lead to altered availability of the corresponding protein, thus affecting various cellular functions. An early indication of gene expression changes can facilitate early and timely management of diseased condition or risk of any abnormal conditions in the body. The present invention relates to identification of a category of virulence factors produced by the resident microbial communities (within or on the human body) called 'Nucleomodulins' (NMs) and utilization of the same for indicating host gene expression changes. The microbial communities, referred to as 'microbiome', form an indispensable part of the human body. There is a well-established link between the human microbiome and the overall health of the host. Symbiotic relationship between host and a balanced diverse microbial community aid in various crucial bodily functions such as immune regulation, micronutrient synthesis, metabolic breakdown drugs, etc. However, imbalances in the microbial compositions, also known as dysbiosis, leading to disturbances in these functions can contribute to the pathogenesis of diseases/disorders. Microbiome possess an intrinsic ability to alter host gene expression by synthesis of metabolites, direct interaction with immune cells, synthesis of effector proteins that interact with host cell receptors thereby changing the functionality of multitude of pathways. The present invention relates to a method for indicating host (human) gene expression changes through identification of a category of microbial effector proteins called `NMs'.

NMs are bacterial effector proteins with an ability to translocate to host nucleus, resulting in alteration of crucial cellular functions. Some of the molecular mechanisms hindered by NMs involve DNA degradation, DNA methylation, histone modifications such as acetylation, methylation leading to epigenetic rewiring, alterations in physical state of nuclei, modulations of transcriptional networks via chromatin remodeling, etc. State of art methods for identification of NMs involves high resolution liquid chromatography coupled to MS (LC-MS/MS) or peptide mass mapping using matrix-assisted laser desorption/ionization-time of flight (MALDI-TOF/TOF), ELISA (Enzyme linked immuno-sorbent assay), microarray method or any other method of analyzing and quantitating the proteins. However, these methods are cumbersome and involves highly specialized skills for the performance of such methods. Also, homology among protein sequences cannot be utilized which can save the time and efforts in terms of generating protein sequences already available in a knowledgebase.

### SUMMARY

Embodiments of the present disclosure present technological improvements as solutions to one or more of the above-mentioned technical problems recognized by the inventors in conventional systems. For example, in one embodiment, a method of identifying biological markers indicative of altered gene expression in an host to combat pathogenic diseases. The method includes obtaining a biological sample from the host. The biological sample is obtained from one or more tissue biopsy sample, oral swab, blood sample, intestinal fluid, cerebrospinal fluid, urine sample, or fecal sample, intestinal swab, intestinal tissue, intestinal fluid according to an affected body part of the host. The method further includes isolating a cellular fraction from the biological sample. The cellular fraction is performed using well-established laboratory techniques. The method further includes extracting a plurality of proteins from the cellular fraction. The proteins from samples can be extracted employing suitable laboratory accepted methods preferably comprising appropriate isotonic buffers followed by centrifugation that will allow cell organelle fractionation and subsequent protein extraction. This can be performed using commercially available kits such as Millipore compartmental protein extraction kit. The method further includes obtaining microbial protein sequence from the plurality of proteins. The microbial proteins are distinguished from the eukaryotic proteins specific to the host and can be isolated by the well-established laboratory techniques. The method further includes scanning a NMs knowledgebase (NMKB) against the plurality of microbial protein sequence; wherein the NMKB comprises of a plurality of pre-defined set of NMs , and wherein the pre-defined set of NMs belong to one or more pathogens associated with one or more disease conditions; and wherein scanning involves similarity matching of amino acid sequence of the plurality of microbial protein sequence with that of pre-defined set of NMs of the NMKB. The method further includes finding the presence of `nuclear localization signal' (NLS) on the plurality of microbial protein sequence. Nuclear localization signals (NLS) are generally short peptides that act as a signal fragment that mediates the transport of proteins from the cytoplasm into the nucleus. The method further includes deriving a "sequence similarity score" of the NLS using a plurality of sequence-homology based proprietary tools and algorithms; and wherein identification of NMs among the plurality of microbial protein sequence possessing NLS is based on pre-set criteria includes: (a) The 'sequence similarity score' of the `nuclear localization signal (NLS)' of the NMs should satisfy a predefined threshold value. A 'higher' sequence similarity score indicates a higher probability of any identified NMs to possess a `nuclear localization signal' having very similar protein sequence to that of a 'known' `nuclear localization signal', wherein the NLS similarity indicated similar mode of action of the identified microbial protein sequence to that of a 'known' nuclear targeting protein; (b) The identified `nuclear localization signal' is exclusively present in a pathogenic microbe and absent in homologs of the NMs in a commensal microbe. The method further includes identifying NMs and the associated pathogen from the NMKB wherein the NMs are the biological markers indicative of altered gene expression in an host. The present disclosure further involves targeting the one or more identified NMs by a delivery construct comprising a biological therapeutic compound (biologicals) wherein the identified NMs are indicative of altered gene expression.

In another aspect, a system for identifying biological markers indicative of altered gene expression in an host to combat pathogenic diseases. The system includes at least one memory storing programmed instructions; one or more Input /Output (I/O) interfaces; and one or more hardware processors, a NMs identification model, operatively coupled to a corresponding at least one memory, wherein the system is configured to obtain a biological sample from the host. The biological sample is obtained from one or more tissue biopsy sample, oral swab, blood sample, intestinal fluid, cerebrospinal fluid, urine sample, or fecal sample, intestinal swab, intestinal tissue, intestinal fluid according to the affected body part of the host. Further, the system is configured to prepare, via the one or more hardware processors, a cellular fraction from the biological sample. The cellular fraction is performed using well-established laboratory techniques. Further, the system is configured to extracting a plurality of proteins from the cellular fraction. The proteins from samples can be extracted employing suitable laboratory accepted methods preferably comprising appropriate isotonic buffers followed by centrifugation that will allow cell organelle fractionation and subsequent protein extraction. This can be performed using commercially available kits such as millipore compartmental protein extraction kit. Further, the system is configured to obtain, via the one or more hardware processors microbial protein sequence from the plurality of proteins. The microbial proteins are distinguished from the eukaryotic proteins specific to the host and can be isolated by the well-established laboratory techniques. Further, the system is configured to scan, via the one or more hardware processors a NMs knowledgebase (NMKB) against the plurality of microbial protein sequence; wherein the NMKB comprises of a plurality of pre-defined set of NMs, and wherein the pre-defined set of NMs belong to one or more pathogens associated with one or more disease conditions; and wherein scanning involves similarity matching of amino acid sequence of the plurality of microbial protein sequence with that of pre-defined set of NMs of the NMKB. Further, the system is configured to find, via the one or more hardware processors the presence of `nuclear localization signal' (NLS) on the plurality of microbial protein sequence. Nuclear localization signals (NLS) are generally short peptides that act as a signal fragment that mediates the transport of proteins from the cytoplasm into the nucleus. Further, the system is configured to derive, via the one or more hardware processors a "sequence similarity score" of the NLS using a plurality of sequence-homology based proprietary tools and algorithms; and wherein identification of NMs among the plurality of microbial protein sequence possessing NLS is based on pre-set criteria includes: (a) The ` sequence similarity score' of the `nuclear localization signal (NLS)' of the NMs should satisfy a predefined threshold value. A 'higher' sequence similarity score indicates a higher probability of any identified NMs to possess a `nuclear localization signal' having very similar protein sequence to that of a 'known' `nuclear localization signal', wherein the NLS similarity indicated similar mode of action of the identified microbial protein sequence to that of a 'known' nuclear targeting protein; (b) The identified `nuclear localization signal' is exclusively present in a pathogenic microbe and absent in homologs of the NMs in a commensal microbe. Further, the system is configured to identify, via the one or more hardware processors, NMs and the associated pathogen from the NMKB wherein the NMs are the biological markers indicative of altered gene expression in an host. The present disclosure further involves targeting the one or more identified NMs by a delivery construct comprising a biological therapeutic compound (biologicals) wherein the identified NMs are indicative of altered gene expression.

In yet another aspect, a computer program product including a non-transitory computer-readable medium having embodied therein a computer program for identifying biological markers indicative of altered gene expression in an host to combat pathogenic diseases. The computer readable program, when executed on a computing device, causes the computing device to obtain a biological sample from the host. The biological sample is obtained from one or more tissue biopsy sample, oral swab, blood sample, intestinal fluid, cerebrospinal fluid, urine sample, or fecal sample, intestinal swab, intestinal tissue, intestinal fluid according to the affected body part of the host. The computer readable program, when executed on a computing device, causes the computing device to prepare, via the one or more hardware processors, a cellular fraction from the biological sample. The cellular fraction is performed using well-established laboratory techniques. The computer readable program, when executed on a computing device, causes the computing device to extract a plurality of proteins from the cellular fraction. The proteins from samples can be extracted employing suitable laboratory accepted methods preferably comprising appropriate isotonic buffers followed by centrifugation that will allow cell organelle fractionation and subsequent protein extraction. This can be performed using commercially available kits such as Millipore compartmental protein extraction kit. The computer readable program, when executed on a computing device, causes the computing device to obtain, via the one or more hardware processors microbial protein sequence from the plurality of proteins. The microbial proteins are distinguished from the eukaryotic proteins specific to the host and can be isolated by the well-established laboratory techniques. The computer readable program, when executed on a computing device, causes the computing device to scan, via the one or more hardware processors a NMs knowledgebase (NMKB) against the plurality of microbial protein sequence; wherein the NMKB comprises of a plurality of pre-defined set of NMs, and wherein the pre-defined set of NMs belong to one or more pathogens associated with one or more disease conditions; and wherein scanning involves similarity matching of amino acid sequence of the plurality of microbial protein sequence with that of pre-defined set of NMs of the NMKB. The computer readable program, when executed on a computing device, causes the computing device to find, via the one or more hardware processors the presence of `nuclear localization signal' (NLS) on the plurality of microbial protein sequence. Nuclear localization signals (NLS) are generally short peptides that act as a signal fragment that mediates the transport of proteins from the cytoplasm into the nucleus. The computer readable program, when executed on a computing device, causes the computing device to derive a "sequence similarity score" of the NLS using a plurality of sequence-homology based proprietary tools and algorithms; and wherein identification of NMs among the plurality of microbial protein sequence possessing NLS is based on pre-set criteria includes: (a) The 'sequence similarity score' of the `nuclear localization signal (NLS)' of the NMs should satisfy a predefined threshold value. A 'higher' sequence similarity score indicates a higher probability of any identified NMs to possess a `nuclear localization signal' having very similar protein sequence to that of a 'known' `nuclear localization signal', wherein the NLS similarity indicated similar mode of action of the identified microbial protein sequence to that of a 'known' nuclear targeting protein; (b) The identified `nuclear localization signal' is exclusively present in a pathogenic microbe and absent in homologs of the NMs in a commensal microbe. The computer readable program, when executed on a computing device, causes the computing device to identify NMs and the associated pathogen from the NMKB wherein the NMs are the biological markers indicative of altered gene expression in an host. The present disclosure further involves targeting the one or more identified NMs by a delivery construct comprising a biological therapeutic compound (biologicals) wherein the identified NMs are indicative of altered gene expression.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates a schematic block diagram of a system for identification of NMs according to some embodiments of the present disclosure.
FIG. 2 is a functional block diagram of the system 100 depicting a plurality of processes for the identification of Nucleomodulins (NMs) according to some embodiments of the present disclosure.
FIGS. 3A and 3B are an exemplary flow diagram for a method of identification of NMs, in accordance with some embodiments of the present disclosure.
FIGS. 4A and 4B shows a flowchart for creating a knowledgebase 'NMDB' according to an embodiment of the present disclosure.
FIG. 5 depicts knowledgebase prepared by collecting disease, associated pathogens, associated NMs and their functions, according to some embodiments of the present disclosure.
FIG. 6 illustrates a flowchart depicting the method for identification of NMs in a diseased condition and targeting the same towards managing the disease, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

As used herein, the term `host' refers to a living organism. In an embodiment, 'host' may refer to human, animal or plant in which a pathogenic infection may be observed.

As used herein, the terms `Nucleomodulins (NMs)' or 'NMs' refer to the effector proteins synthesized by the bacteria which can enter the host cell nucleus. NMs interact with host nuclear proteins thereby interfering with host cellular pathways which lead to changes in gene expression patterns providing survival advantage to the pathogen.

As used herein, the term `microbial effector proteins' refers to the proteins synthesized by microbes which are delivered to the host cells using but not limited to secretion systems. Once, inside the host cell these proteins aid in interfering with numerous host cellular processes thereby impacting the host cell.

As used herein, the term 'nuclear localization signal' refers to the small stretch of amino acids that facilitates entry of proteins (NMs) to the nucleus of the host cell.

As used herein, the term 'commensal' refers to microbes/microbes which are considered beneficial to the host or cause no harm to the host.

As used herein, the term `pathogenic microbes' or 'pathogens' refers to microbes which cause a disease in host.

As used herein, the term `human microbiome' refers to the community of microorganisms such as bacteria, fungi, viruses etc. residing within and on the human body. Bacteria forming a part of this microbiome are responsible for regulation of various cellular mechanisms of human host.

As used herein, the term `taxonomic abundance' refers to the occurrence/ abundance of taxonomic groups within a microbiome sample which can be collected from any site of the body. The taxonomic abundance can be obtained as genus, species, or strain taxonomic level in the form of the matrix comprising the abundances with the name of taxa as rows and samples at columns. Certain genes like 16S rRNA in bacteria are used as marker genes for understanding phylogeny of taxonomic groups present in a microbiome sample.

As used herein, the term 'dysbiosis' refers to imbalance in the microbiome that led to an increase in the abundance of pathogens thereby hindering beneficial functional capabilities of commensals. Dysbiosis can contribute to the development/progression of a variety of diseases.

As used herein, the term `gene expression' refers to a process in which information stored in DNA (Deoxyribonucleic acid) is converted to functional gene products. The first major step in this process involves transcription followed by translation. In eukaryotes transcription takes place in the nucleus. Any internal or external factors can cause changes in gene expression wherein the activity or level of expression of a specific gene product or a group of gene products is altered.

As used herein, the term `transcription' refers to the process of synthesizing RNA molecules from DNA sequence.

As used herein, the term `homologous proteins' refers to proteins sharing a common evolutionary ancestor, thus having similar protein sequences.

As used herein, the term expression `functional domains of protein' refers to regions within the protein molecule which are inherently stabilizing and can thus form independent functional units that perform a distinct function. A protein can be comprised of one or several functional domains.

As used herein, the term `protein annotation' refers to the process of identifying and determining the peculiar functions and properties of the protein based on various computational analysis.

As used herein, the term 'Microbiome data' refers to nucleotide sequence of the genome of the microbes isolated from any sample. The `microbiome data' can be obtained in form of 'reads' which are short stretches of nucleotide sequences. In one case, the `reads can correspond to the genes encoding 16S ribosomal RNA (16S rRNA) and can be called as `16S rRNA data'. In another case, the 'reads' can correspond to the whole genomes of the microbes.

NMs (NMs) are bacterial effector proteins with an ability to translocate to host nucleus, resulting in alteration of crucial cellular functions. Some of the molecular mechanisms hindered by NMs involve DNA degradation, DNA methylation, histone modifications such as acetylation, methylation leading to epigenetic rewiring, alterations in physical state of nuclei, modulations of transcriptional networks via chromatin remodeling, etc. The mechanisms utilized by NMs to target nucleus include mimicking eukaryotic cells by possessing a nuclear localization sequence (NLS) or via diffusion through nuclear pores depending on the size of the protein. Apart from these, the mechanism of translocation of some of the NMs are poorly characterized and need further investigations. Such examples include HRgpA of Porphyromonas gingivalis, OrfX of Listeria monocytogenes, Cif of Escherichia coli, etc. One of the widely discussed strategy for evading nuclear membrane by NMs is utilization of short amino acid motifs called 'NLSs' (Nuclear Localization Signals). NLSs are broadly categorized into two main classes namely, classical NLS (cNLS) and non-classical NLS (ncNLS) which are recognized by importin α or importin β respectively depending on the composition of the motif. Importins refer to a class of 'karyopherins' which import the NLS tagged NMs inside the nucleus. The cNLS is rich in positively charged amino acids such as lysine and arginine and is further divided into 2 classes, monopartite and bipartite. As the name suggests, monopartite NLS is composed of single short stretch of 4-8 basic amino acids whereas bipartite NLS is made up of two such sequences which are linked by a spacer sequence. ncNLS does not comprise of any specific amino acids as observed in the case of cNLS. Among ncNLS, proline-tyrosine category of NLS has been reported in recent literature.

Early reports of bacterial effector proteins targeting host nucleus include Agrobacterium tumefaciens, a plant pathogen responsible for causing crown tumor disease in plants. Since then, it has been routinely used in genetic engineering of plants. Among human pathogens, NMs have been reported in Helicobacter pylori, Mycobacterium tuberculosis, Legionella pneumophila, Acinetobacter baumannii, Escherichia coli, Klebsiella pneumoniae, Porphyromonas gingivalis, etc. For example, four NMs (Rv0256c, Rv1988, Rv2966c and Rv3423) have been studied in Mycobacterium tuberculosis, causative agent of tuberculosis, which is also notoriously known to exhibit a range of host immune response evading strategies. These NMs have been reported to be majorly involved in suppressing genes implicated in inhibiting action of macrophages, thereby providing intracellular survival advantage to the pathogen. Another example includes a set of transposases and outer membrane proteins from Acinetobacter baumannii, a bacteria frequently associated with nosocomial infections. Two NMs from the bacteria namely, AbOmpA and Tnp comprise of a classical monopartite NLS that helps in targeting nucleus. Once in the nucleus AbOmpA hinders physiological state of the cell causing cell death. Tnp, on the other hand, suppresses E-cadherin expression by inducing DNA methylation in CpG islands of promoter region of the certain genes. Further, A DNA methylase HsdM (which is a subunit of type 1 restriction modification system), secreted by Klebsiella pneumoniae, has been experimentally proven to be translocated to host nucleus with the help of monopartite NLS sequence `7-KKAKAKK-13' recognized by eukaryotic importin alpha protein. Such bacterial effector proteins with the ability to translocate to the host nucleus and subsequently resulting in disease related events like inhibition of immune function, cell death etc, thus can be probed for early identification of possible host gene expression changes. This, in turn, can aid in prevention, early diagnosis and better therapeutic management of diseases. The present method and system relate to identification of microbial effector proteins referred to as 'NMs (NM)' which have the ability to translocate to the nucleus of host cell, thereby influencing host gene expression related to a diseased condition. In the present disclosure, identification of NMs involves analysis of biological samples that include (i) isolation of nuclear proteins from the biological sample obtained from the host and (ii) detection of NMs listed in a predefined knowledgebase called 'NMDB' (database of NMs).

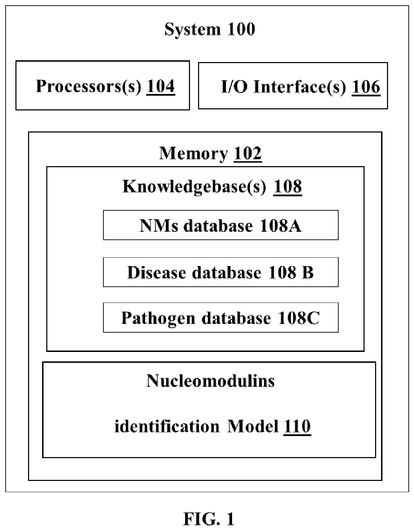

Referring now to the drawings, and more particularly to FIG. 1 through FIG. 7, where similar reference characters denote corresponding features consistently throughout the figures, there are shown preferred embodiments, and these embodiments are described in the context of the following exemplary system and/or method.

FIG. 1 illustrates a schematic block diagram of a system for identification of NMs according to some embodiments of the present disclosure. The system 100 indicates host (human) gene expression changes through identification of a category of microbial effector proteins called `NMs (NMs)'. The system 100 annotates these NMs for their functions and properties based on various computational analysis. It should be appreciated that analysis using any other model is well within the scope of this disclosure.

In an embodiment, the system 100 includes one or more processors 104, communication interface device(s) or input/output (I/O) interface(s) 106, and one or more data storage devices or memory 102 operatively coupled to the one or more processors 104. The one or more processors 104 that are hardware processors can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and `hardware processors' may be used interchangeably. In an embodiment, the system 100 can be implemented in a variety of mobile computing systems, such as mobile devices, laptop computers, notebooks, hand-held devices, workstations, mainframe computers, servers, a network cloud and the like. The I/O interface (s) 106 may include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface(s) 106 can include one or more ports for connecting a number of devices to one another or to another server. The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 102 may include a database or repository. The memory 102 may comprise information pertaining to input(s)/output(s) of each step performed by the processor(s) 104 of the system 100 and methods of the present disclosure. In an embodiment, the database may be external (not shown) to the system 100 and coupled via the I/O interface 106. The memory 102, further include NMs identification model 110. The NMs identification model 110 performs analysis on proteins extracted from the host to detect pre-defined set of NMs. When the pre-defined set of NMs are present in a knowledgebase designed according to the method disclosed in the system, the NMs can be identified and an associated pathogen as well as associated disease can be identified. However, when the set of NMs are not present in the knowledgebase, the protein sequence specific to NMs is identified by 16s rRNA analysis and further annotation is performed using computational analysis according to the method disclosed in the present invention for identifying the associated pathogen and the associated disease. In another embodiment, the protein sequence specific to NMs is identified by performing whole proteome sequencing and further annotation is performed using computational analysis according to the method disclosed in the present invention for identifying the associated pathogen and the associated disease. In an embodiment, the memory 102 may include a knowledgebase or a database or a repository 108. The memory 102 may comprise information pertaining to input(s)/output(s) of each step performed by the processor(s) 104 of the system 100 and methods of the present disclosure. In an embodiment, the knowledgebase may be external (not shown) to the system 100 and coupled via the I/O interface 106. In an embodiment, the memory 102 includes NMs database 108A, disease database 108B and pathogen database 108C. The knowledgebase 108 comprising NMs database 108A, disease database 108B and pathogen database 108C is referred by the system 100 each time a pre-defined set of NMs is input to identify whether the pre-defined set of protein sequence of NMs has existed in the knowledgebase 108, an assigns a first method of analysis. When the pre-defined set of NMs does not find any reference in the knowledgebase, then a second method of analysis or a third method of analysis is assigned by the system 100 to identify the protein sequence of the NMs. The memory 102 further includes a plurality of modules (not shown here) comprises programs or coded instructions that supplement applications or functions performed by the system 100 for executing different steps involved in the process of identifying NMs. The plurality of modules, amongst other things, can include routines, programs, objects, components, and data structures, which perform particular tasks or implement particular abstract data types. The plurality of modules may also be used as, signal processor(s), node machine(s), logic circuitries, and/or any other device or component that manipulates signals based on operational instructions. Further, the plurality of modules can be used by hardware, by computer-readable instructions executed by the one or more hardware processors 104, or by a combination thereof. The plurality of modules can include various sub-modules (not shown).

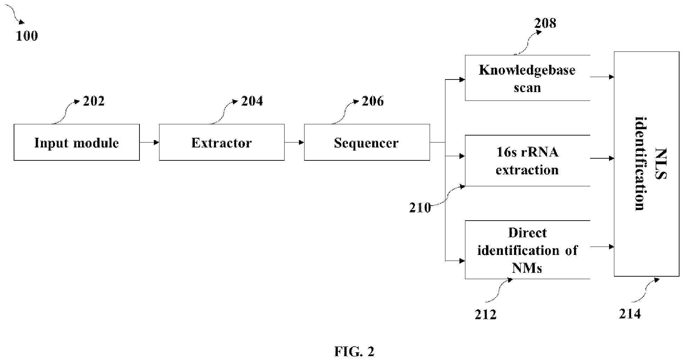

FIG. 2 is a functional block diagram of the system 100 depicting a plurality of processes for the identification of NMs according to some embodiments of the present disclosure.

As illustrated in FIG. 2, the NMs identification model handles input protein sequence in multitude of ways to identify NMs. At step 202, input module of the system 100 receives a biological sample from the host. The biological sample includes one or more tissue biopsy sample, oral swab, blood sample, intestinal fluid, cerebrospinal fluid, urine sample, or fecal sample. The sample extraction depends on the site involved as an affected area or the type of disease that suggest from where the sample to be collected from. In case of skin infections, skin lesion biopsies can be used to collect layers of skin with the help of a sterile razor or blade. The type of skin biopsy to be performed such as shave, incisional, punch or excisional will depend on the severity of the infection and as suggested by the dermatologist. In another embodiment if the focal point of infection is some internal organs such as intestines, kidneys, pancreas, stomach, etc., then tissue samples can be obtained using suitable biopsy procedures. In one embodiment, if the site of infection is stomach, then apart from tissue sample, fluid sample can also be obtained using gastric aspiration wherein a thin tube is passed through the nose or mouth into the stomach to collect the fluid. Fecal samples can also be used in case of gastrointestinal infections. In cases where the infection is not detected in the fecal sample, samples can be obtained through an endoscopic biopsy of the GI tract. If the central nervous system is affected in any of the conditions, then cerebrospinal fluid (CSF) can be used as a sample or brain tissue sample can be collected using computed tomography guided aspiration, which is a method involving a needle biopsy. In another embodiment, in the case of but not limited to hematological disorders, blood samples can be collected using a sterile needle. In yet another embodiment, if the site of infection is in lung, then one of the following methods such as bronchial brushing, transbronchial biopsy, bronchoalveolar lavage (BAL), thoracentesis, etc., can be utilized. In yet another embodiment, in case of infection in the upper respiratory tract sample collection from lung can be performed by oropharyngeal (OP) and nasopharyngeal (NP) swabs and sputum collection. Urine samples may also be obtained in situations involving urinary infections or occasionally gastrointestinal infections. Sterile swabs can be used to collect saliva samples in case of oral diseases or other diseases in which oral microbiome is altered. The input module 202 can facilitate multiple communications within a wide variety of networks and protocol types , including wired networks , for example, LAN, cable , etc . , and wireless networks , such as WLAN , cellular , or satellite . The input module 202 may include one or more ports for connecting a number of devices including assistive technology devices or adaptive products used by people with disability to one another or to another server . The biological sample received from the input module 202 is utilized to extract nuclear protein using the extractor 204. Once the input module 202 receives the sample, it processes the sample to the extractor 204. The extractor 204, performs extraction of the nuclear protein. The nuclear proteins from samples can be extracted employing suitable laboratory accepted methods preferably comprising of appropriate isotonic buffers followed by centrifugation that will allow cell organelle fractionation and subsequent protein extraction. This can be performed using commercially available kits such as millipore compartmental protein extraction kit to extract nuclear proteome. Any other 'accepted' method to extract nuclear protein is within the scope of the invention. The sequencer 206 identifies the sequence of the extracted protein. The sequencing is performed using high throughput sequencing techniques. The sequencing results in the generation of a plurality of protein sequences. The protein sequence of the nuclear protein is then subjected to a plurality of processes that handles the nuclear protein sequence to finally detect the NMs present and further identification of the associated disease and the pathogen involved. The identification of one or more NMs from the protein extract of the host by selecting one among a plurality of processes for identifying one or more NMs from the protein extract of the host involves:
(a) A first process 208, comprises of scanning the one or more NMs identified in the protein extract of the host against a knowledgebase comprising a plurality of pre-defined set of NMs wherein the NMs belongs to one or more pathogens associated with one or more disease conditions; and wherein scanning involves similarity matching of amino acid sequences of the NMs with that of NMs of the knowledgebase. The one or more pre-defined set of NMs is scanned against the knowledgebase 'NMDB' wherein the 'NMDB' comprises of a plurality of pre-defined set of NMs wherein the NMs belongs to one or more pathogens associated with one or more disease conditions. The scanning involves similarity matching of amino acid sequences of the NMs with that of NMs of the KB and finding the presence of `nuclear localization signal' (NLS) on the protein.
(b) A second process 210, comprises of isolating 16s rRNA from the protein extract. The sequence is directed to 16s rRNA extraction. The 16s rRNA data represents the microbiome data of one of the plurality of diseases caused due to probable influence of the NMs and is obtained from public repositories. In the second process 210, the sequence is taken for whole protein extraction for direct identification of proteins using high resolution liquid chromatography coupled to MS (LC-MS/MS) or peptide mass mapping using matrix-assisted laser desorption/ionization-time of flight (MALDI-TOF/TOF) can be utilized to identify and quantify NMs listed in Set_{NM} . In another embodiment, for blood samples, extracted protein samples can be analyzed using ELISA (Enzyme linked immuno sorbent assay) wherein Nm specific antibodies can be used as capture agents for detection of NMs of interest. If the number of NMs for a given disease is higher, then microarray can be used to detect multiple NMs. Each immobilized spot-on microarray will correspond to different probes specific to target NMs sequences enabling simultaneous detection of various NMs implicated in selected disease. Any other method of analyzing and quantitating the proteins depending on the type of samples such as biofluids or tissue samples are within the scope of the invention.
(c) A third process 212, comprises of extracting proteome sequence from the biological sample; and identifying NMs if present in the proteome sequence.

Once, the one or more NMs are identified from the protein extract of the host by selecting one among a plurality of processes disclosed above, nuclear localization signal (NLS) 214 is identified using pre-set conditions. A sequence similarity score of the NLS is calculated using a plurality of sequence-homology based proprietary tools and algorithms.

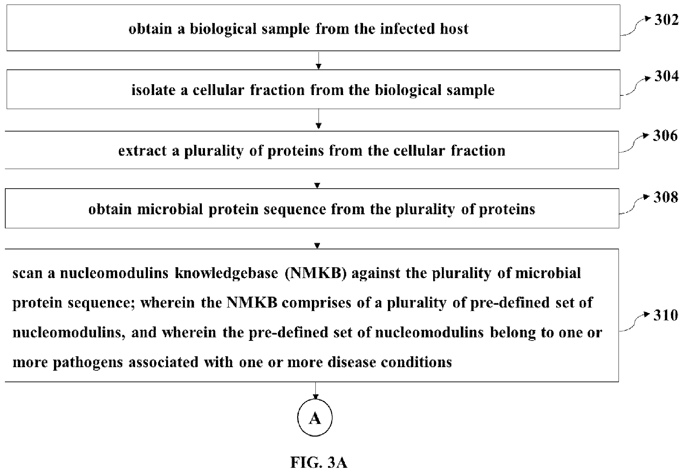

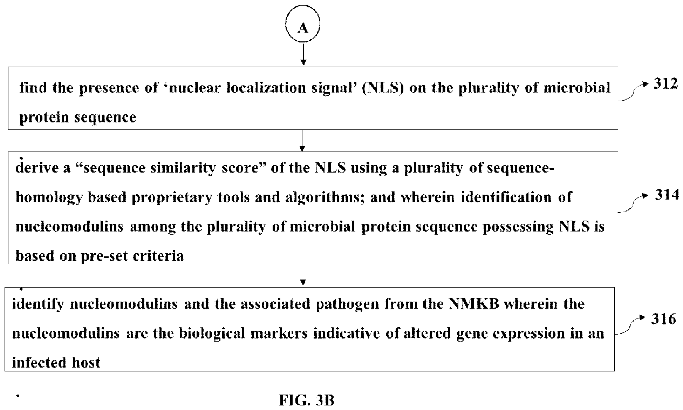

FIGS. 3A and 3B are an exemplary flow diagram for a method of identification of NMs, in accordance with some embodiments of the present disclosure.

In an embodiment, the system 100 comprises one or more data storage devices or the memory 102 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the system 100 as depicted in FIG. 1 through FIG. 7. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

At step 302 of the method 300, the one or more hardware processors 104 are configured to obtain a biological sample from the host. The input module of system 100 receives the biological sample. The biological sample may include one or more of tissue biopsy sample, oral swab, blood sample, intestinal fluid, cerebrospinal fluid, urine sample, or fecal sample. The method for extracting samples depends largely on the site and type of a disease. In case of skin infections, skin lesion biopsies can be used to collect layers of skin with the help of a sterile razor or blade. The type of skin biopsy to be performed such as shave, incisional, punch or excisional will depend on the severity of the infection and as suggested by the dermatologist. In another embodiment if the focal point of infection is some internal organs such as intestines, kidneys, pancreas, stomach, etc., then tissue samples can be obtained using suitable biopsy procedures. In one embodiment, if the site of infection is stomach, then apart from tissue sample, fluid sample can also be obtained using gastric aspiration wherein a thin tube is passed through the nose or mouth into the stomach to collect the fluid. Fecal samples can also be used in case of gastrointestinal infections. In cases where the infection is not detected in the fecal sample, samples can be obtained through an endoscopic biopsy of the GI tract. If the central nervous system is affected in any of the conditions, then cerebrospinal fluid (CSF) can be used as a sample or brain tissue sample can be collected using computed tomography guided aspiration, which is a method involving a needle biopsy. In another embodiment, in the case of but not limited to hematological disorders, blood samples can be collected using a sterile needle. In yet another embodiment, if the site of infection is in lung, then one of the following methods such as bronchial brushing, transbronchial biopsy, bronchoalveolar lavage (BAL), thoracentesis, etc., can be utilized. In yet another embodiment, in case of infection in the upper respiratory tract sample collection from lung can be performed by oropharyngeal (OP) and nasopharyngeal (NP) swabs and sputum collection. Urine samples may also be obtained in situations involving urinary infections or occasionally gastrointestinal infections. Sterile swabs can be used to collect saliva samples in case of oral diseases or other diseases in which oral microbiome is altered.

At step 304 of the method 300, the one or more hardware processors 104 are configured to isolate a cellular fraction from the biological sample. In an embodiment, the cellular fractionation separates subcellular components and organelles, so that the structures, functions, and molecular compositions of isolated components may be studied. The process starts by homogenizing tissue in a buffered isotonic solution. Other known methods for cell fractionation involves differential centrifugation, density-equilibrium centrifugation, affinity chromatography, free-flow electrophoresis (FFE), affinity purification, fluorescence-activated organelle sorting (FAOS), microfluidic technology and the like.

At step 306 of the method 300, the one or more hardware processors 104 are configured to extract a plurality of proteins from the cellular fraction. Nuclear proteins from samples can be extracted employing suitable laboratory accepted methods preferably comprising of appropriate isotonic buffers followed by centrifugation that will allow cell organelle fractionation and subsequent protein extraction. This can be performed using commercially available kits such as Millipore compartmental protein extraction kit to extract nuclear proteome. Any other 'accepted' method to extract nuclear protein is within the scope of the invention. At step 308 of the method 300, the one or more hardware processors 104 are configured to obtain microbial protein sequence from the plurality of proteins. The microbial proteins are distinguished from the eukaryotic proteins specific to the host and can be isolated by the well-established laboratory techniques. At step 310 of the method 300, the one or more hardware processors 104 are configured to scan a NMs knowledgebase (NMKB) against the plurality of microbial protein sequence. The NMKB comprises of a plurality of pre-defined set of NMs, and wherein the pre-defined set of NMs belong to one or more pathogens associated with one or more disease conditions. Scanning the one or more NMs identified in the protein extract of the host against a knowledgebase comprising a plurality of pre-defined set of NMs wherein the NMs belongs to one or more pathogens associated with one or more disease conditions; and wherein scanning involves similarity matching of amino acid sequences of the NMs with that of NMs of the knowledgebase. The one or more pre-defined set of NMs is scanned against the NMs knowledgebase wherein the `NMKB' comprises of a plurality of pre-defined set of NMs wherein the NMs belongs to one or more pathogens associated with one or more disease conditions. The scanning involves similarity matching of amino acid sequences of the NMs with that of NMs of the KB and finding the presence of `nuclear localization signal' (NLS) on the protein. At step 312 of the method 300, the one or more hardware processors 104 are configured to find, via the one or more hardware processors, the presence of `nuclear localization signal' (NLS) on the plurality of microbial protein sequence. Nuclear localization signals (NLS) are generally short peptides that act as a signal fragment that mediates the transport of proteins from the cytoplasm into the nucleus. This NLS-dependent protein recognition, a process necessary for cargo proteins to pass the nuclear envelope through the nuclear pore complex, is facilitated by members of the importin superfamily. Unlike proteins bound to the endoplasmic reticulum or mitochondria, whose N-terminal targeting signals are often cleaved after arrival at their destination organelle, nuclear localization signals remain intact and can be located at almost any part of the protein sequence, indicating the possibility of multiple rounds of nucleocytoplasmic transport. In recent years, NLS are widely used in cancer treatment and viral infection prevention, and researchers paid more attention to identifying novel NLS motifs and the import nucleoporins that recognize and bind them. In the present disclosure, NLS are identified as the biological markers indicating altered gene expression. At step 314 of the method 300, the one or more hardware processors 104 are configured to derive a "sequence similarity score" of the NLS using a plurality of sequence-homology based proprietary tools and algorithms; and wherein identification of NMs among the plurality of microbial protein sequence possessing NLS is based on pre-set criteria. The pre-set criteria involves:
(i) The `sequence similarity score' of the `nuclear localization signal (NLS)' of the NMs should satisfy a predefined threshold value. A 'higher' sequence similarity score indicates a higher probability of any identified NMs to possess a `nuclear localization signal' having very similar protein sequence to that of a 'known' `nuclear localization signal', wherein the NLS similarity indicated similar mode of action of the identified microbial protein sequence to that of a 'known' nuclear targeting protein.
(ii) The identified `nuclear localization signal' is exclusively present in a pathogenic microbe and absent in homologs of the NMs in a commensal microbe.

At step 316 of the method 300, the one or more hardware processors 104 are configured to identify NMs and the associated pathogen from the NMKB wherein the NMs are the biological markers indicative of altered gene expression in an host.

In an embodiment of the present invention, there are other methods that can be utilized in identifying microbial protein sequence without scanning the NMKB, such as (a) 16s rRNA extraction, and (b) whole proteome sequencing. In the 16s rRNA extraction, process starts with the collection of the biological sample. The 16S rRNA sequencing (one type of Next Generation Sequencing (NGS) involves identifying from the sequencing data, resident microbes which are potential pathogen (this information to be derived from literature). This step provides novel information on 'disease/infection-pathogen' association. Further, protein sequence files corresponding to the identified pathogenic microbes are obtained from one of widely accepted public repositories such as, National Centre of Biotechnology Information (NCBI) and finding the presence of `nuclear localization signal' (NLS) on the protein. The other sequence repository utilized for obtaining protein sequence include Uniprot, InterPro, Protein Information Resource (PIR) , swissprot, TrEMBL, RCSB-PDB, SCOP, CATH, Prosite, Mint and KEGG. The identification of NLS follows the same steps disclosed in the steps 312 and 314 the involves, querying a `protein sequence' for identification of the NLS sequence using a plurality of `sequence homology' based proprietary tools and algorithms and then identifying NMs based on pre-set criteria specified in the step 314 wherein the ` sequence similarity score' of the `nuclear localization signal (NLS)' of the NMs should satisfy a predefined threshold value. The first pre-set criteria being a 'higher' sequence similarity score that indicates a higher probability of any identified NMs to possess a `nuclear localization signal' having very similar protein sequence to that of a 'known' `nuclear localization signal'. This, in turn, may indicate similarity in the mode of action of any identified NMs to that of a 'known' nuclear targeting protein. And another pre-set criteria being `nuclear localization signal' to be exclusively present in pathogenic microbe. In the whole proteome sequencing, the biological sample is collected, and proteome extraction is performed on the microbial cells by the well-established methods. The protein sequences is obtained through state-of-art methods such as, 'mass spectrometry'. Further, the process includes finding the presence of `nuclear localization signal' (NLS) on the protein. The identification of NLS follows the same steps disclosed in the steps 312 and 314 the involves, querying a `protein sequence' for identification of the NLS sequence using a plurality of `sequence homology' based proprietary tools and algorithms and then identifying NMs based on pre-set criteria specified in the step 314 wherein the 'sequence similarity score' of the `nuclear localization signal (NLS)' of the NMs should satisfy a predefined threshold value. The first pre-set criteria being a 'higher' sequence similarity score that indicates a higher probability of any identified NMs to possess a `nuclear localization signal' having very similar protein sequence to that of a 'known' `nuclear localization signal'. This, in turn, may indicate similarity in the mode of action of any identified NMs to that of a 'known' nuclear targeting protein. And another pre-set criteria being `nuclear localization signal' to be exclusively present in pathogenic microbe.

In an embodiment of the present disclosure, the method further includes targeting the one or more identified NMs by a delivery construct comprising a biological therapeutic compound (biologicals) wherein the identified NMs are indicative of altered gene expression. The biological therapeutic compound is selected from the group comprising of one or more siRNA (small interfering RNAs) and shRNAs (short hairpin RNAs) that specifically binds mRNA of one or more NMs, CRISPR with a construct including gRNA (guide RNA) complementary to the gene of interest and CRISPR-associated protein 9 (Cas9) nuclease, and Proteolysis targeting chimeras (PROTACs) that promote ubiquitination and subsequent degradation of the target NMs by employing cellular endogenous proteasomal system. According to an embodiment of the present invention, targeting one or more of the identified NMs involves RNA interference that are used to target nucleomodulins expression in specific cells or tissues by delivering siRNA (small interfering RNAs) or shRNAs (short hairpin RNAs). Both these RNAs are designed to specifically bind mRNA of one or more NMs. In case of siRNA, RISC (RNA-induced silencing complex) binds with it resulting in generation of guide strand that will target the nucleomodulins mRNA of interest whereas shRNA is processed within the cells to generate siRNAs that can bind to the target nucleomodulins mRNA. Binding of these RNAs to target nucleomodulins mRNA results in its translational inhibition. In another approach, CRISPR interference is used. gDNA designed in accordance with the nucleomodulins of interest with reference to the backend database. It comprises of sequence complementary to nucleomodulins and a transactivating CRISPR RNA (tracrRNA) sequence that helps guide the CRISPR-associated protein 9 (Cas9) nuclease to the target site. Upon binding, this complex will induce double stranded breaks that will render a non-functional protein that is unable to access the host nucleus. In another embodiment, protein degradation systems such as `Proteolysis targeting chimeras (PROTACs)' can also be used. These systems will promote ubiquitination and subsequent degradation of the target nucleomodulins by employing cellular endogenous proteasomal system. Other methods of targeting proteins in vivo are within the scope of this disclosure. Further, after a pre-defined time, effectiveness of these techniques in downregulating or silencing the expression of target nucleomodulins can be evaluated using techniques such as but not limited to Western blotting, immunostaining etc.

In the embodiment of the present disclosure, the input module 202 of the system 100 obtains the biological sample of the host at two-time stamps, a time stamp 1 and a time stamp 2. The time stamp 1 is considered as a time when the biological sample is collected from the host before administration of nuclear constructs targeting NMs for suppression/ downregulation of NMs. The time stamp 2 is considered as a time when the biological sample is collected from the host after administration of nuclear constructs targeting NMs for suppression/ downregulation of NMs at an interval based on a pre-specified time. The significance of differences between the levels of NMs at the two-time stamps can be assessed using statistical methods such as parametric, non-parametric tests, machine learning based algorithms including decision trees etc. The decrease in the amount of NMs after the administration of the nuclear constructs indicate effectiveness of the adapted treatment regime.

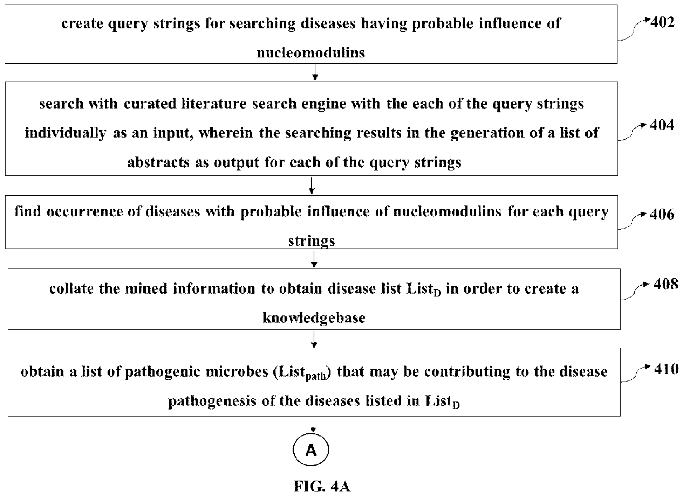

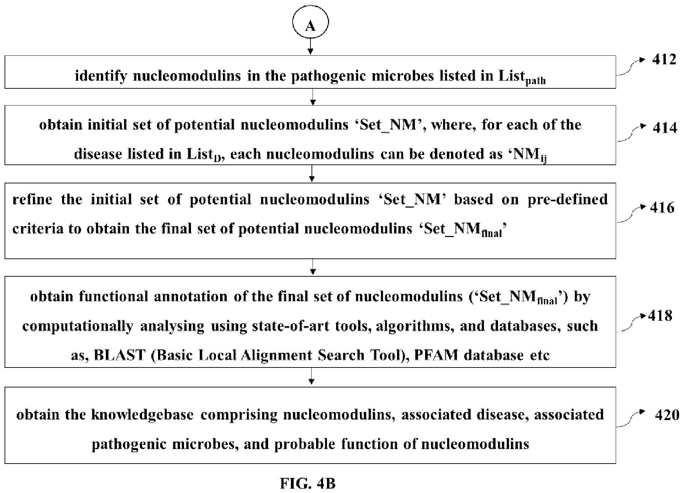

FIGS. 4A and 4B shows a flowchart for creating a knowledgebase 'NMDB' according to an embodiment of the present disclosure.

As illustrated in FIGS. 4A-4B, the system 100 is further configured to create knowledgebase 'NMDB'. Initially at step 402, query strings are created for searching diseases having probable influence of NMs. At the step 404, search is performed using the search strings created in the previous step. The search is carried on curated literature search engine with the each of the query strings individually as an input, wherein the searching results in the generation of a list of abstracts as output for each of the query strings. The type of query strings utilized in the identification of relevant literature includes Q = [Microbiome OR Metagenome OR Microbiota] AND [`host gene expression' OR `gene expression'] AND [modified OR change], is used as an input against literature search engines which are repositories for scientific literature and may include but are not limited to Pubmed, Pubtator, etc. This process resulted in a set of 'abstracts' which were then manually curated to obtain a final `disease list'. The manual curation also took into consideration the prevalence of the diseases globally. At the step 406, occurrence of diseases with probable influence of NMs for each query strings is identified. At the step 408, the mined information is collated to obtain disease list List_{D} in order to create a knowledgebase. The final list (List_{D}) comprised of diseases such as but not limited to colitis, tuberculosis, colorectal cancer, hepatocellular carcinoma, atopic dermatitis, etc. At the step 410, a list of pathogenic microbes (Listₚₐₜₕ) is obtained that may be contributing to the disease pathogenesis of the diseases listed in List_{D}. In order to obtain a list of pathogenic microbes that may be contributing to the disease pathogenesis of the diseases listed in List_{D}, the following steps were performed, (a) download microbiome data (16S rRNA data) corresponding to the diseases listed in 'List_{D}' from public repositories; and (b) computationally analyze 16S rRNA data of each disease list in List_{D} to obtain a list of pathogenic microbes (Listₚₐₜₕ) that are comparatively higher in abundance in diseased state than in healthy state. At the step 412, NMs are identified in the pathogenic microbes listed in Listₚₐₜₕ. Identification of NMs in the pathogenic microbes listed in Listₚₐₜₕ was performed through the following steps: (a) Protein sequence files corresponding to the microbes listed in Listpath obtained from the repository National Centre of Biotechnology Information (NCBI); and (b) computationally analyze, `nuclear localization signal' to identify potential NMs. The obtained `protein sequence' data was computationally analyzed using publicly available state-of-art as well as proprietary tools/algorithms that utilizes sequence-based information of `nuclear localization signal' to identify potential NMs. At the step 414, initial set of potential NMs 'Set_NM' is obtained, where, for each of the disease listed in List_{D}, each NMs can be denoted as 'NMij. This step resulted in the initial set of potential NMs 'Set_NM', where, for each of the disease listed in List_{D}, each NMs can be denoted as 'NMij', wherein, 'i' ranges from '1' to 'the total number of pathogenic microbes identified for a particular disease' and 'j' ranges from '1' to 'the total number of NMs identified for a particular pathogenic microbe'. In addition, the analysis also provided a 'sequence similarity score' for each of the NMs 'NMᵢⱼ'. At the step 416, the initial set of potential NMs 'Set_NM' is refined based on pre-defined criteria to obtain the final set of potential NMs 'Set_NM_{final}'. The predefined criteria are as follows:
(i) The 'sequence similarity score' of the `nuclear localization signal (NLS)' of the NMs should satisfy a predefined threshold value. A 'higher' sequence similarity score indicates a higher probability of any identified NMs 'NMᵢⱼ' to possess a `nuclear localization signal' having very similar protein sequence to that of a 'known' `nuclear localization signal'. This, in turn, may indicate similarity in the mode of action of any identified NMs 'NMᵢⱼ' to that of a 'known' nuclear targeting protein.
(ii) Any identified NMs 'NMᵢⱼ' should be exclusively present in pathogenic microbe `i'. When utilizing these NMs for targeted therapy, it would be important to focus on proteins exclusively present in pathogens. This would lead to higher precision by minimizing the chance of causing disturbance in the resident commensal community. Furthermore, it would also result in minimizing potential side effects which would have otherwise caused interruptions in the proper functioning of commensals thereby inducing unfavorable changes.
(iii) In case of presence of any homolog of any NMs 'NMᵢⱼ' in commensal microbe, the `nuclear localization signal (NLS)' should be absent in the corresponding protein sequence. Most of the microbial NMs utilize eukaryotic nuclear localization signal to translocate to host nucleus. For example, IgA protease from Neisseria meningitidis with an exclusive functional NLS present only when it has been isolated from hyperinvasive species. Once inside the host nucleus, IgA protease mediates nuclear cleavage of p65/RelA thereby inducing early NF-κB activation, subsequently resulting in apoptosis. IgA protease from non-invasive species was unable to cleave p65/RelA due to the absence of NLS. Thus, a homolog of any NMs 'NMij' lacking any NLS would be unable to translocate to host nucleus and affect host gene expression.
(iv) The selected NMs should not have any homologous proteins in the host. This would aid in designing target specific strategies without compromising on host cellular functioning.

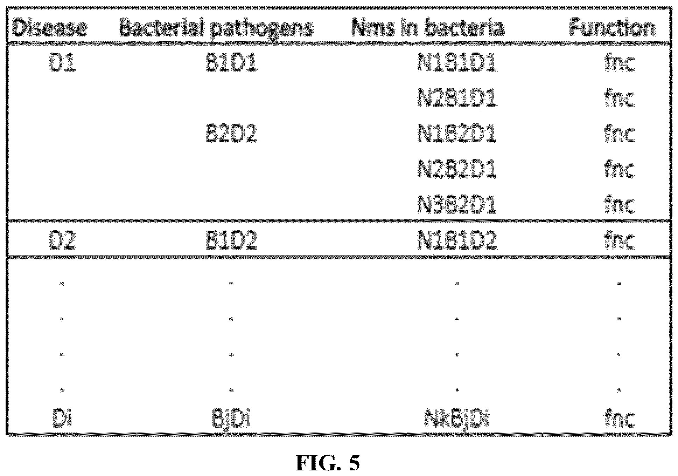

At the step 418, the functional annotation of the final set of NMs ('Set_NMfinal') is performed by computationally analysing using state-of-art tools, algorithms, and databases, such as, BLAST (Basic Local Alignment Search Tool), PFAM database etc. In order to obtain details about the probable function of the NMs, the corresponding protein sequences were computationally analyzed using state-of-art tools, algorithms, and databases, such as, BLAST (Basic Local Alignment Search Tool), PFAM database etc. Utilization of any other widely accepted tools, packages and databases for functional annotation of proteins are within the scope of the current invention. At the step 420, the knowledgebase 'NMDB' comprising NMs, associated disease, associated pathogenic microbes, and probable function of NMs is obtained. The 'NMDB' (database of NMs) created by collating the details obtained in steps 402-418 which comprises of details related to disease, pathogenic microbes, NMs and their probable function and is represented in FIG. 5.

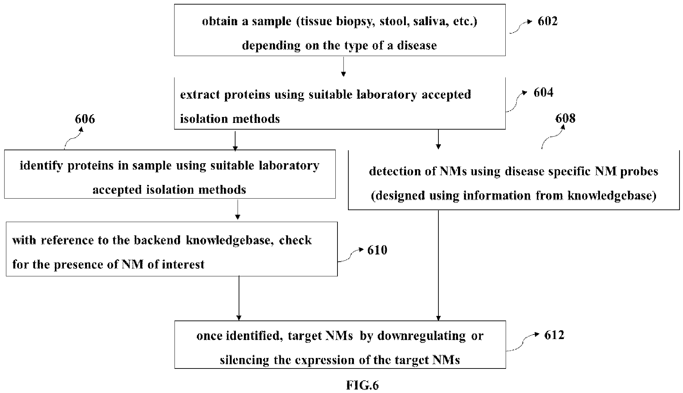

FIG. 6 illustrates a flowchart depicting the method for identification of NMs in a diseased condition and targeting the same towards managing the disease, according to some embodiments of the present disclosure.

As Illustrated in the FIG. 6, end-to-end flow of managing disease state by targeting NMs identified from the specified pathogen is disclosed. The disease management starts by obtaining a sample at 602. The biological sample is collected from the site of infection or as per disease protocol. The biological sample may be a tissue biopsy, stool, saliva, blood, other body fluid and the like. Once biological sample is obtained, it is further processed to extract nuclear proteins using suitable laboratory accepted isolation methods at 604. Some of the known methods for nuclear protein extraction involves direct identification of proteins using high resolution liquid chromatography coupled to MS (LC-MS/MS) or peptide mass mapping using matrix-assisted laser desorption/ionization-time of flight (MALDI-TOF/TOF), ELISA (Enzyme linked immuno sorbent assay), microarray-based analysis and the like. Any other method of analyzing and quantitating the proteins depending on the type of samples such as biofluids or tissue samples are within the scope of the invention. Further analysis of NMs follows a bifurcation in adopting wet-lab methods (i.e. bio-chemical methods) and dry-lab methods (i.e. computational analysis). At 606, the proteins are identified in the sample using suitable laboratory accepted isolation methods. At 608, NMs are detected using specific NMs probes that are designed using information from the knowledgebase. At 610, the proteins are identified in the sample using suitable laboratory accepted isolation methods is scanned against reference knowledgebase to check the presence of NMs of interest. Once NMs are identified at 612, they can be targeted using methods comprising of protein degradation molecules designed in accordance with NM sequences. The NMs thus identified are further targeted by suitable methods to control alteration in gene expression. The suitable methods includes either downregulating the expression of target NMs or silencing the expression of target NMs. To ascertain effectiveness in downregulation/ silencing the NMs, presence of NMs are further evaluated in the biological sample of the host after a pre-defined period of time. In case, NMs are found in the biological sample, a molecular construct of target NMs can be re-administered to execute complete downregulation/ silencing.

According to an embodiment of the present invention, a device for predicting altered gene expression is disclosed.

The device for predicting altered gene expression comprises:
an input module for receiving the biological sample;
a processor configured to analyze the input sample using the methods of the present disclosure,
wherein the processor further comprising:
   a module for identifying NMs, and
   a module for predicting pathogen associated with the identified NMs; and
   an output module for confirming the affected state of the host based on the analysis of the processor.

The input module receives the biological sample as obtained from one or more tissue biopsy sample, oral swab, blood sample, intestinal fluid, cerebrospinal fluid, urine sample, or fecal sample, intestinal swab, intestinal tissue, intestinal fluid according to an affected body part of the host. The processor is configured to analyze the biological sample using the NMs identification model 110. The analysis involves utilizing one of the three methods of identification of NMs disclosed in the present disclosure wherein, the methods include:
(i) The first process comprises of scanning the one or more NMs identified in the protein extract of the host against the knowledgebase, NMKB. The scanning is performed to identify a plurality of pre-defined set of NMs wherein the NMs belongs to one or more pathogens associated with one or more disease conditions; and wherein scanning involves similarity matching of amino acid sequences of the NMs with that of NMs of the knowledgebase.
(ii) The second process comprises of isolating 16s rRNA from the protein extract. The sequence is directed to 16s rRNA extraction. The 16s rRNA data represents the microbiome data of one of the plurality of diseases caused due to probable influence of the NMs and is obtained from public repositories.
(iii) The third process comprises of extracting proteome sequence from the biological sample; and identifying NMs if present in the proteome sequence.

The processor further comprises of several specialized modules for performing NMs identification. The module for identifying NMs involves identification of nuclear localization signals (NLS). The module for predicting pathogen associated with the identified NMs utilizes a plurality of tools and algorithms specialized in ascertaining the correlation of expressed NMs and a causative pathogen using sequence similarity scoring techniques. The processor further comprises of an output module for confirming the exposed state of the host based on the analysis of the processor. The output module reads the NLS. finding the presence of nuclear localization signal (NLS) on the proteome sequence and derive the sequence similarity score of the NLS using a plurality of sequence-homology based proprietary tools and algorithms, wherein microbial protein sequence identification within the proteome sequence is based on pre-set criteria specified above in the present disclosure.

### USE CASE:

A case study for obtaining a list of pathogenic microbes (Listpath) that may be contributing to the disease pathogenesis of the diseases listed in ListD (as per some of the embodiments described above) and identification of NMs in the pathogenic microbes listed in 'Listpath' is presented below:

One of the diseases in the knowledgebase 'NMDB' corresponds to colorectal cancer, which is the second most common cancer worldwide. colorectal cancer has been characterized by complex interplay between aberrant genetic and epigenetic modifications that lead to tumorigenesis. There are various reports linking changes in microbiome dynamics which are concurrent with alterations in genetic and epigenetic changes. In order to identify the pathogenic microbes associated to colorectal cancer, microbiome data (microbial taxonomic abundance files) corresponding to tumor tissue samples of colorectal cancer patients and normal tissue samples are downloaded using Google's BigQuery platform. Analysis of microbiome data corresponding to any other type of samples such as stool, saliva etc. are within the scope of the present invention. The downloaded `microbiome data' are analyzed through widely accepted publicly available as well as proprietary tools/algorithms to obtain a list of pathogenic microbes (Listpath) that are comparatively higher in abundance in diseased state than in healthy state. In this particular case study, bacterial groups with significantly higher abundance in tumor tissue samples as compared to the normal tissue samples are selected for further analysis and included in 'Listpath'. In another embodiment, other microbes such as virus etc. can also be considered. One of the bacterial groups, thus identified, is Fusobacterium nucleatum (Fn). Numerous studies involving mice with colon cancer xenograft have demonstrated reduction in tumor growth and proliferation on treating with antibiotics against Fusobacterium nucleatum. On transfection of CRC cell lines with Fn, it significantly elevated global DNA methylation of tumor suppressing genes involving methyltransferases which are previously known to initiate carcinogenesis such as, DNMT1 and DNMT3a. These studies provide a comprehensive idea regarding epigenetic rewiring of host genome by Fn to facilitate tumor progression. Apart from Fusobacterium nucleatum, other bacterial species which are identified to have significantly higher abundance in colon tumor tissue samples include, Escherichia coli, Enterococcus faecalis, Streptococcus gallolyticus, and Bacteroides fragilis. These bacteria are also included in 'Listₚₐₜₕ'. Once the 'Listₚₐₜₕ' is curated for the disease `colorectal cancer', in order to identify NMs from these bacteria, `protein sequence files' corresponding to each entry of 'Listₚₐₜₕ' are downloaded from National Centre of Biotechnology Information (NCBI) repository. The obtained `protein sequence' data is computationally analyzed using publicly available state-of-art as well as proprietary tools/algorithms that utilizes sequence-based information of `nuclear localization signal' to identify potential NMs. This initial set of NMs were further filtered based on the predefined criteria as described above to obtain the final set of NMs in Fusobacterium nucleatum are presented in Table-1.

**Table-1**

| **NMs** | | **Primary function** | **Probable role in gene expression alterations** |
|---|---|---|---|
| **Ribosomal proteins** | | Involved in protein synthesis | >Ribosomal proteins can be involved in activating or upregulating expression of certain pro- affecting cascade of cell signaling pathways involved in silencing oncoproteins. |
| 1. 50S ribosomal protein L34 | | | |
| 2. 30S ribosomal protein S9 | | | |
| 3. 50S ribosomal protein L20 | | | |
| 4. 30S ribosomal protein S13 | | | |
| 5. 50S ribosomal protein L15 | | | |
| 6. 50S ribosomal protein L32 | | | >These proteins hold the ability to perform extrinsic ribosomal tasks by interacting with certain transcriptional regulators thereby disturbing and interfering with optimal functioning/assembly of eukaryotic ribosomal proteins. These interactions may further lead to faulty synthesis of crucial tumor suppressing genes. |
| 7. 50S ribosomal protein L2 | | | |
| 8. 30S ribosomal protein S11 | | | |
| **DNA/RNA-binding proteins** | | Binds to DNA or RNA depending upon the binding domain thereby regulating range of cellular processes. | > Plays a role in attaching to DNA at different locations, resulting in conformational alterations that influence gene expression factors. |
| 9. RNA helicase | | | |
| 10. DNA helicase UvrC | | | |
| 11. terminase | | | |
| 12. excinuclease ABC subunit A | | | >Dysregulation or overexpression of helicases can lead to genomic instability and promote the growth and survival of cancer cells. The role of helicases in tumor progression can vary depending on the specific helicases. |
| **Mobile elements** | | Enzymes with the ability to incorporate mobile genetic elements at specific genomic sites. | > Integration of mobile genetic elements into the genome may cause changes in gene expression including silencing of crucial tumor suppressing genes. |
| | 13. Transposases | | |
| **Transferases** | | Transfer of specific groups hereby altering or adding to functionality of molecule | > DNA methylase adds methyl moieties to DNA. The resulting methylation led to regulation of gene expression, chromatin structure, and numerous other cellular processes by determining the accessibility of specific regions of DNA to transcription factors and other regulatory proteins. |
| 14. cobalamin adenosyltransferase | | | |
| 15. DNA methylase | | | |
| 16. dephospho-CoA kinase | | | |
| 17. shikimate dehydrogenase | | | |
| 18. rubrerythrin | | | |
| 19. 4-hydroxy-3-methylbut-2-enyl diphosphate reductase | | | |
| | | | > Phosphorylation of proteins by kinases influences catalytic capabilities of the protein or makes it available for binding with other proteins thereby positively regulating pathways that favor tumorigenesis. |
| **Transporters** | | Transport of proteins and other molecules | > Transporters often carry proteins around them such as GTPases into the nucleus that can directly interfere with various cell cycling pathways, thereby inducing tumorigenesis. |
| 20. ABC transporter | | | |
| 21. electron transporter RnfB | | | |
| 22. copper-transporting ATPase | | | |
| 23. iron ABC transporter | | | |
| 24. MFS transporter | | | > May participate in signal transduction, modulating gene expression via secondary messengers. Their function also encompasses metabolite transport, affecting the expression of genes involved in cellular metabolism. |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The present invention relates to a method for indicating gene expression changes in the host through identification of a category of microbial effector proteins called `NMs (NMs)'. NMs (NMs) are bacterial effector proteins with an ability to translocate to host nucleus, resulting in alteration of crucial cellular functions. Identification and targeting the NMs could help predicting altered gene expression even before the NMs actually damages the DNA of the host. The method involves constructing and utilizing a knowledgebase comprising NMs identified through curated literature search engines known to cause altered gene expression. Subsequently, utilizing the computational analysis according to the method of present disclosure, the identified NMs are mapped to the associated diseases and the associated pathogens causing the disease. Finally, functional annotation of the NMs is performed by computationally analysing the corresponding protein sequences. The knowledgebase is utilized as a first process of handling the pre-defined set of NMs once received by the input module of the NMs identification model. Once the pre-defined set of NMs are received by the input module, the NMs identification model scans against the knowledgebase to identify NMs, associated disease, associated pathogen and the function. The present invention also suggest suppression/ downregulation of gene by utilizing nuclear construct targeting the NMs.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A method (300) of identifying biological markers indicative of altered gene expression in a host with disease associated gut dysbiosis, the method comprising steps:
obtaining (302), a biological sample from the host;
isolating (304), a cellular fraction from the biological sample;
extracting (306), a plurality of proteins from the cellular fraction;
obtaining (308), microbial protein sequence from the plurality of proteins;
scanning (310), via one or more hardware processors, a nucleomodulins knowledgebase (NMKB) against the plurality of microbial protein sequence; wherein the NMKB comprises of a plurality of pre-defined set of nucleomodulins (NMs), and wherein the pre-defined set of NMs belong to one or more pathogens associated with one or more disease conditions; and wherein scanning involves similarity matching of amino acid sequence of the plurality of microbial protein sequence with that of pre-defined set of NMs of the NMKB;
finding (312), via the one or more hardware processors, the presence of 'nuclear localization signal' (NLS) on the plurality of microbial protein sequence;
deriving (314), via the one or more hardware processors, a sequence similarity score of the NLS using a plurality of sequence-homology based proprietary tools and algorithms, and wherein identification of NMs among the plurality of microbial protein sequence possessing NLS is based on pre-set criteria comprising:
(a) the sequence similarity score of the nuclear localization signal (NLS) of the NMs should satisfy a predefined threshold value. A higher sequence similarity score indicates a higher probability of any identified NMs to possess a NLS having very similar protein sequence to that of a known NLS, wherein the NLS similarity indicated similar mode of action of the identified microbial protein sequence to that of the known nuclear targeting protein, and
(b) the identified NLS is exclusively present in a pathogenic microbe and absent in homologs of the NMs in a commensal microbe; and identifying (316), via the one or more hardware processors, NMs and the associated pathogen from the NMKB, wherein the NMs are the biological markers indicative of altered gene expression in an host.

2. The method as claimed in claim 1, wherein the biological sample is obtained from one or more tissue biopsy sample, oral swab, blood sample, intestinal fluid, cerebrospinal fluid, urine sample, or fecal sample, intestinal swab, intestinal tissue, intestinal fluid according to a affected body part of the host.

3. The method as claimed in claim 1, wherein obtaining the microbial protein sequence without scanning the NMKB comprises steps:
isolating 16s rRNA from the obtained sample;
identifying one or more species/genera comprising highest similarity match with the isolated 16s rRNA, wherein the identified species/genera have been reported as pathogens in literature;
obtaining a proteome sequence of the identified genus/species from a plurality of sequence repository; and
identifying the NMs in the proteome sequence, wherein the identification of NMs involves:
finding the presence of nuclear localization signal (NLS) on the proteome sequence and deriving the sequence similarity score of the NLS using a plurality of sequence-homology based proprietary tools and algorithms, wherein microbial protein sequence identification within the proteome sequence is based on pre-set criteria specified in claim 1.

4. The method as claimed in claim 1, wherein obtaining the microbial protein sequence without scanning the NMKB comprises steps:
extracting proteome sequence from the biological sample; and
identifying the NMs in the proteome sequence wherein the identification of NMs involves:
finding the presence of NLS on the proteome sequence and deriving sequence similarity score of the NLS using a plurality of sequence-homology based proprietary tools and algorithms; and
wherein microbial protein sequence identification within the proteome sequence is based on pre-set criteria specified in claim 1.

5. The method as claimed in claim 3, wherein the plurality of sequence repository is selected from National Centre for Bioinformatics (NCBI), Uniprot, InterPro, Protein Information Resource (PIR) , swissprot, TrEMBL, RCSB-PDB, SCOP, CATH, Prosite, Mint and KEGG.

6. The method as claimed in claim 1, wherein the NMKB is constructed using the steps:
(i) preparing disease list (List_{D}) comprising a plurality of diseases by:
(a) executing, knowledge mining on a plurality of scientific databases of diseases to identify a plurality of diseases having a probable influence of NMs in the disease etiology, wherein the knowledge mining is executed using a query string Q = [Microbiome OR Metagenome OR Microbiota] AND [`host gene expression' OR `gene expression'] AND [modified OR change];
(b) collating the abstracts fetched by the string Q;
(c) screening manually, the abstracts to identify diseases captured; and
(d) preparing the List_{D};
(ii) preparing a list of pathogenic microbes (Listₚₐₜₕ) by identifying pathogenic microbes associated with the plurality of diseases listed in ListD, wherein preparing the Listₚₐₜₕ includes:
(a) obtaining microbiome data (16S rRNA data) corresponding to the plurality of diseases listed in List_{D} from public repositories;
(b) analysing the microbiome data through a plurality of tools and algorithms to obtain the Listₚₐₜₕ wherein the pathogens listed in Listₚₐₜₕ have higher abundance in diseased state than in healthy state; and
(c) manually curating the Listₚₐₜₕ for improving accuracy through literature mining; and
(iii) identifying NMs in Listₚₐₜₕ by:
(a) obtaining protein sequence files corresponding to the pathogen listed in Listₚₐₜₕ from a protein sequence the repository;
(b) analyzing computationally, the protein sequence data of organisms listed in Listₚₐₜₕ wherein the protein sequence data utilizes nuclear localization signal to identify an initial set of potential NMs (Set_NM) and the corresponding sequence similarity score; and
(c) refining the initial set of potential NMs (Set_NM) comprising a plurality of NMs (NMᵢⱼ) associated with the plurality of diseases listed in List_{D} to obtain a final set of potential NMs (Set_NM_{final}) based on a set of pre-defined criteria.

7. The method as claimed in claim 6, wherein the set of pre-defined criteria comprising:
(a) the sequence similarity score of the organism satisfies a distinct threshold value to indicate similarity in the mode of action of the identified NMs (NMᵢⱼ) to that of a known nuclear targeting protein,
(b) identified NMᵢⱼ to be exclusively present in the organism (i),
(c) identified homolog of the NMᵢⱼ is deprived of corresponding nuclear localization signal, and
(d) identified NMᵢⱼ does not have any homologous proteins in the host.

8. The method as claimed in claim 1, wherein the method further involves targeting the one or more identified NMs by a delivery construct comprising a biological therapeutic compound (biologicals) wherein the identified NMs are indicative of altered gene expression, and wherein the biological therapeutic compound is selected from the group comprising of one or more siRNA (small interfering RNAs) and shRNAs (short hairpin RNAs) that specifically binds mRNA of one or more NMs, CRISPR with a construct including gRNA (guide RNA) complementary to the gene of interest and CRISPR-associated protein 9 (Cas9) nuclease, and proteolysis targeting chimeras (PROTACs) that promote ubiquitination and subsequent degradation of the target NMs by employing cellular endogenous proteasomal system.

9. A device for predicting altered gene expression, comprising :
an input module for receiving the biological sample;
a processor configured to analyze the input sample using the method performed in any of the claim 1, claim 3 and claim 4,
wherein the processor further comprising:
a module for identifying NMs, and
a module for predicting pathogen associated with the identified NMs; and
an output module for confirming the affected state of the host based on the analysis of the processor.

10. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
obtain a biological sample from the host;
isolate a cellular fraction from the biological sample;
extract a plurality of proteins from the cellular fraction;
obtain microbial protein sequence from the plurality of proteins;
scan via one or more hardware processors, a NMs knowledgebase (NMKB) against the plurality of microbial protein sequence; wherein the NMKB comprises of a plurality of pre-defined set of NMs (NMs), and wherein the pre-defined set of NMs belong to one or more pathogens associated with one or more disease conditions; and wherein scanning involves similarity matching of amino acid sequence of the plurality of microbial protein sequence with that of pre-defined set of NMs of the NMKB;
find via the one or more hardware processors, the presence of nuclear localization signal (NLS) on the plurality of microbial protein sequence;
derive via the one or more hardware processors, a sequence similarity score of the NLS using a plurality of sequence-homology based proprietary tools and algorithms, and wherein identification of NMs among the plurality of microbial protein sequence possessing NLS is based on pre-set criteria comprising:
(a) the sequence similarity score of the NLS of the NMs should satisfy a predefined threshold value. A higher sequence similarity score indicates a higher probability of any identified NMs to possess a NLS having very similar protein sequence to that of a known NLS, wherein the NLS similarity indicated similar mode of action of the identified microbial protein sequence to that of the known nuclear targeting protein, and
(b) the identified NLS is exclusively present in a pathogenic microbe and absent in homologs of the NMs in a commensal microbe; and
(c) identify via the one or more hardware processors, NMs and the associated pathogen from the NMKB, wherein the NMs are the biological markers indicative of altered gene expression in an host.

11. The system as claimed in claim 10, wherein the NMKB is constructed using the steps:
(i) preparing disease list (List_{D}) comprising a plurality of diseases by:
(a) executing, knowledge mining on a plurality of scientific databases of diseases to identify a plurality of diseases having a probable influence of NMs in the disease etiology, wherein the knowledge mining is executed using a query string Q = [Microbiome OR Metagenome OR Microbiota] AND [`host gene expression' OR `gene expression'] AND [modified OR change];
(b) collating the abstracts fetched by the string Q;
(c) screening manually, the abstracts to identify diseases captured; and
(d) preparing the List_{D};
(ii) preparing a list of pathogenic microbes (Listpath) by identifying pathogenic microbes associated with the plurality of diseases listed in ListD, wherein preparing the Listpath includes:
(a) obtaining microbiome data (16S rRNA data) corresponding to the plurality of diseases listed in List_{D} from public repositories;
(b) analysing the microbiome data through a plurality of tools and algorithms to obtain the Listₚₐₜₕ wherein the pathogens listed in Listₚₐₜₕ have higher abundance in diseased state than in healthy state; and
(c) manually curating the Listₚₐₜₕ for improving accuracy through literature mining;
and
(iii) identifying NMs in Listpath by:
(a) obtaining protein sequence files corresponding to the pathogen listed in Listₚₐₜₕ from a protein sequence the repository,
(b) analyzing computationally, the protein sequence data of organisms listed in Listₚₐₜₕ wherein the protein sequence data utilizes nuclear localization signal to identify an initial set of potential NMs (Set NM) and the corresponding sequence similarity score, and
(c) refining the initial set of potential NMs (Set_NM) comprising a plurality of NMs (NMᵢⱼ) associated with the plurality of diseases listed in List_{D} to obtain a final set of potential NMs (Set_NM_{final}) based on a set of pre-defined criteria.

12. The system as claimed in claim 10, wherein the set of pre-defined criteria comprising:
(a) the sequence similarity score of the organism satisfies a distinct threshold value to indicate similarity in the mode of action of the identified NMs (NMᵢⱼ) to that of a known nuclear targeting protein,
(b) identified NMᵢⱼ to be exclusively present in the organism (i),
(c) identified homolog of the NMᵢⱼ is deprived of corresponding nuclear localization signal, and
(d) identified NMᵢⱼ does not have any homologous proteins in the host.

13. One or more non-transitory machine-readable information storage mediums comprising one or more instructions which when executed by one or more hardware processors cause:
obtaining, a biological sample from the host;
isolating, a cellular fraction from the biological sample;
extracting, a plurality of proteins from the cellular fraction;
obtaining, microbial protein sequence from the plurality of proteins;
scanning, a nucleomodulins knowledgebase (NMKB) against the plurality of microbial protein sequence; wherein the NMKB comprises of a plurality of pre-defined set of nucleomodulins (NMs), and wherein the pre-defined set of NMs belong to one or more pathogens associated with one or more disease conditions, and wherein scanning involves similarity matching of amino acid sequence of the plurality of microbial protein sequence with that of pre-defined set of NMs of the NMKB;
finding, the presence of 'nuclear localization signal' (NLS) on the plurality of microbial protein sequence;
deriving, a sequence similarity score of the NLS using a plurality of sequence-homology based proprietary tools and algorithms, and wherein identification of NMs among the plurality of microbial protein sequence possessing NLS is based on pre-set criteria comprising:
(a) the sequence similarity score of the nuclear localization signal (NLS) of the NMs should satisfy a predefined threshold value. A higher sequence similarity score indicates a higher probability of any identified NMs to possess a NLS having very similar protein sequence to that of a known NLS, wherein the NLS similarity indicated similar mode of action of the identified microbial protein sequence to that of the known nuclear targeting protein, and
(b) the identified NLS is exclusively present in a pathogenic microbe and absent in homologs of the NMs in a commensal microbe; and
identifying, NMs and the associated pathogen from the NMKB, wherein the NMs are the biological markers indicative of altered gene expression in an host.

14. The one or more non-transitory machine-readable information storage mediums as claimed in claim 13, wherein the NMKB is constructed using the steps:
(i) preparing disease list (List_{D}) comprising a plurality of diseases by:
(a) executing, knowledge mining on a plurality of scientific databases of diseases to identify a plurality of diseases having a probable influence of NMs in the disease etiology, wherein the knowledge mining is executed using a query string Q = [Microbiome OR Metagenome OR Microbiota] AND [`host gene expression' OR `gene expression'] AND [modified OR change];
(b) collating the abstracts fetched by the string Q;
(c) screening manually, the abstracts to identify diseases captured; and
(d) preparing the List_{D};
(ii) preparing a list of pathogenic microbes (Listpath) by identifying pathogenic microbes associated with the plurality of diseases listed in ListD, wherein preparing the Listpath includes:
(a) obtaining microbiome data (16S rRNA data) corresponding to the plurality of diseases listed in List_{D} from public repositories;
(b) analysing the microbiome data through a plurality of tools and algorithms to obtain the Listₚₐₜₕ wherein the pathogens listed in Listₚₐₜₕ have higher abundance in diseased state than in healthy state; and
(c) manually curating the Listₚₐₜₕ for improving accuracy through literature mining;
and
(iii) identifying NMs in Listₚₐₜₕ by:
(a) obtaining protein sequence files corresponding to the pathogen listed in Listₚₐₜₕ from a protein sequence the repository;
(b) analyzing computationally, the protein sequence data of organisms listed in Listₚₐₜₕ wherein the protein sequence data utilizes nuclear localization signal to identify an initial set of potential NMs (Set_NM) and the corresponding sequence similarity score; and
(c) refining the initial set of potential NMs (Set_NM) comprising a plurality of NMs (NMᵢⱼ) associated with the plurality of diseases listed in List_{D} to obtain a final set of potential NMs (Set_NM_{final}) based on a set of pre-defined criteria.

15. The one or more non-transitory machine-readable information storage mediums as claimed in claim 13, wherein the set of pre-defined criteria comprising:
(a) the sequence similarity score of the organism satisfies a distinct threshold value to indicate similarity in the mode of action of the identified NMs (NMᵢⱼ) to that of a known nuclear targeting protein,
(b) identified NMᵢⱼ to be exclusively present in the organism (i),
(c) identified homolog of the NMᵢⱼ is deprived of corresponding nuclear localization signal, and
(d) identified NMᵢⱼ does not have any homologous proteins in the host.
